# EUROPÄISCHE PATENTSCHRIFT

(11) **EP 1 384 467 B1**
(45) Veröffentlichungstag und Bekanntmachung des Hinweises auf die Patenterteilung: **16.05.2007**
(21) Anmeldenummer: 03014579.1
(22) Anmeldetag: 07.07.2003
(51) Int. Cl.: A61K 8/72, A61Q 5/02, A61K 8/89, A61Q 5/12

(54) **Haarshampoo für feines und fettiges Haar**
Shampoo for fine and greasy hair
Shampooing pour cheveux fins et gras

(30) Priorität: 27.07.2002 DE 10234262
(43) Veröffentlichungstag der Anmeldung: 28.01.2004
(73) Patentinhaber: Beiersdorf AG, 20245 Hamburg (DE)
(72) Erfinder: Albrecht, Harald Dr., 22083 Hamburg (DE); Heitmann, Birgit, 22769 Hamburg (DE)

(56) Entgegenhaltungen:
- WO-A-95/09599
- WO-A-98/18434
- FR-A- 2 712 490
- FR-A- 2 799 971
- US-A- 5 776 444
- US-B1- 6 180 576

## Beschreibung

Die vorliegende Erfindung betrifft ein konditionierendes Haarshampoo für feines und/oder fettiges Haar.

Bei der Körperpflege des Menschen spielt die Haarwäsche eine zentrale Rolle. Die Reinigung der Haare und der Kopfhaut von körpereigenem Fett, Hautabschilferungen, Schmutz und Gerüchen entspricht einem Grundbedürfnis des Menschen.

Zur Befriedigung dieses Bedürfnisses stand dem Menschen bis ins 20.Jahrhundert hinein allein Seife zur Verfügung, die aufgrund ihres alkalischen pH-Wertes für die Kopfhaut und die Augenschleimhäute wenig verträglich war und häufig Ablagerungen von Kalkseifen im Haar zurück ließ. In den dreißiger Jahren des 20,Jahrhunderts erblickte das erste alkylsulfathaltige Shampoo das Licht der Welt Seit Mitte der sechziger Jahre erobern Alkylethersulfate und andere Tenside den Shampoomarkt. Mit ihnen können die Nachteile von seifenhaltigen Zubereitungen vermieden werden. Heutzutage müssen moderne Shampoos das Haar nicht nur reinigen und gut verträglich sein. Vielmehr sollen sie das Haar auch pflegen und seine Frisierbarkeit und optische Attraktivität erhöhen.

Haarshampoos enthalten eine Vielzahl unterschiedlicher Komponenten um den einzelnen Anforderungen an das Produkt gerecht zu werden:

Die Reinigungskraft der Shampoos wird bewirkt durch die Anwesenheit von anionischen, amphoteren und nichtionischen Tensiden als oberflächenaktive Verbindungen in den Zubereitungen. Tenside sorgen darüber hinaus für das Schaumvermögen der Haarreinigungsmittel. Wichtig bei der Auswahl der Tenside ist darüber hinaus ihre Unempfindlichkeit gegenüber der Wasserhärte, ihre biologische Abbaubarkeit, ihre Verträglichkeit mit anderen Komponenten der Zubereitung sowie ihr Preis. Ein viel verwendetes Shampootensid ist beispielsweise Alkylethersulfat.

Darüber hinaus enthalten Shampoos eine Reihe von Konsistenzregulatoren, die der Zubereitung die gewünschte Viskosität verleihen. Diese Verdicker bewirken eine Vergrößerung der Tensidmicellen bzw. eine Quellung der Wasserphase der Zubereitung. Verdicker können aus chemisch sehr unterschiedlichen Stoffklassen gewählt werden. So werden u.a. Elektrolyte (z.B. Natriumchlorid), Alkanolamide (z.B. Fettsäure-Monoethanolamide), niedrig ethoxylierte Fettalkohole (z.B. Diethylenglycolmonolaurylether), hochethoxylierte Ether, Ester und Diester sowie polymere Verdicker eingesetzt. Zu den polymeren Verdickern zählen beispielsweise Celluloseether. Darüber hinaus finden auch Polyacrylate und Hydrokolloide als Verdicker Verwendung. Polymere Verdicker haben den großen Vorteil, dass die durch sie erzeugte Viskosität weitgehend temperaturunabhängig ist.

Neben Parfüm- und Farbstoffen sowie einer Reihe von Verbindungen, welche die Haltbarkeit der Zubereitungen erhöhen, werden in jüngerer Zeit den Haarshampoos unterschiedliche Arten von Wirkstoffen zugefügt. Hierzu zählen. neben UV-Absorbern, Vitaminen oder Pflanzenextrakten auch sogenannte Haarkonditionierer (engl. conditioner), welche das Haar pflegen und seine Kämmbarkeit und seinen Griff verbessern sowie seinen Glanz erhöhen. Konditionierer ziehen, im Gegensatz zu den meisten anderen Bestandteilen von Shampoos, auf das Haar auf und verbleiben dort nach dem Spülen. Sie lagern sich aufgrund ihres Molekülaufbaus an die Schadstellen der Cuticula des Haares und glätten das Haar. Dadurch wird das Haar weniger rauh und spröde, die Frisur bekommt deutlich mehr Glanz und läßt sich leichter kämmen. Auch wird das Haar weniger empfindlich für eine elektrostatische Aufladung. Die wichtigsten haarkonditionierenden Substanzen in Shampoos stellen die polymeren quatären Ammoniumverbindungen dar. Auch können kationische Cellulosederivate und Polysaccharide eingesetzt werden. Weiterhin werden auch Silikonverbindungen zur Konditionierung eingesetzt.

Nachteilig am Stande der Technik ist der Umstand, dass es bisher. nur unzureichend gelungen ist, die Menge des sich auf dem Haar ablagernden Konditionierers zu regulieren. Meist kommt es bei der wiederholten Anwendung von Haarkonditionierern zu einer Zunahme der Menge an Konditionierer auf dem Haar. Durch die Zunahme von Konditionierern auf dem Haar wird dieses mehr und mehr beschwert (engl. build upeffect), die Frisur erscheint ungepflegt und strähnig. Diese Effekte treten insbesondere bei feinem und/oder fettigem Haar auf. Feines Haar ist besonders dünn und elastisch bzw. leicht verbiegbar. Fettiges Haar wird durch das Gewicht des Haarfettes zusätzlich beschwert. Aus diesem Grunde enthalten herkömmliche Haarwaschmittel für feines und/oder fettiges Haar keine oder nur in geringen Mengen Konditionierer. Dadurch werden die Kämm- und Griffeigenschaften des Haares jedoch nur unzureichend verbessert und das Haar erscheint matt und stumpf.

Es war daher die Aufgabe der vorliegenden Erfindung ein Haarwaschmittel zu entwickeln, welches die Kämmbarkeit und das Volumen von feinen und/oder fettigen Haaren erhöht und dessen Griff verbessert, ohne das Haar zu stark zu beschweren.

Überraschend gelöst wird die Aufgabe durch eine Haarreinigungszubereitung enthaltend eine Kombination aus
a) einem oder mehreren Tensiden in einer Konzentration von 5 bis 30 Gewichts-%,
b) einem oder mehreren Filmbildnern in einer Konzentration von 0,01 bis 5 Gewichts-%,
c) einem oder mehreren Elastomeren in einer Konzentration von 0,02 bis 2Gewichts-%, jeweils bezogen auf das Gesamtgewicht der Zubereitung,
neben gegebenenfalls weiteren Wirk-, Hilfs- und Zusatzstoffen.

Die erfindungsgemäßen Zubereitungen verleihen dem Haar Glanz, Spannkraft und Fülle. Die Kämmbarkeit und der Griff sind deutlich verbessert.

Man versteht erfindungsgemäß unter Elastomeren Polymere Verbindungen, die mittels einer Vernetzungsreaktion unter Verwendung von difunktionellen Vernetzungsmolekülen, in eine dreidimensionale Struktur überführt werden.

Die Elastomere werden in einer Konzentration von 0,2 bis 2 Gewichts-%, jeweils bezogen auf das Gesamtgewicht der Zubereitung eingesetzt.

Erfindungsgemäß ist der Einsatz von Dimethicon/Vinyldimethicon Kreuzpolymeren (INCI: DimethiconeNinyl Dimethicone Crosspolymer, z.B. DOW CORNING 9506 Powder®, DOW Corning 9509 Silicone®) als Elastomer.

US-A-5 776 444 offenbart Haarwaschmittel, die quervernetztes Dimethicon enthalten.

Es ist erfindungsgemäß von Vorteil wenn die erfindungsgemäßen Filmbildner in einer Konzentration von 0,01 bis 5 Gewichts-%, bevorzugt in einer Konzentration von 0,05 bis 2 Gewichts-% und ganz besonders bevorzugt in einer Konzentration von 0,01 bis 1 Gewichts-%, jeweils bezogen auf das Gesamtgewicht der Zubereitung eingesetzt werden.

Erfindungsgemäß vorteilhafte Filmbildner können dabei aus den in der Tabelle aufgelisteten Verbindungen gewählt werden.

**Tabelle 1:**

| **Bezeichnung nach INCI** | **CAS-Nummer** | **Polymertyp** | **Beispiel** **(Handelsname)** |
|---|---|---|---|
| Polyquaternium-2 | CAS 63451-27-4 | Urea, N, N'-bis[3-(dimethylamino)propyl]-, polymer mit 1,1'-oxybis(2-chloroethan) | Mirapol® A-15 |
| Polyquaternium-5 | CAS 26006-22-4 | Acrylamid, β-Methacryloxyethyltriethyl ammoniummethosulfat | |
| Polyquaternium-6 | CAS 26062-79-3 | N,N-Dimethyl-N-2-propenyl-2-propenaminiumchlorid | Merquat® 100 |
| Polyquaternium-7 | CAS 26590-05-6 | N,N-Dimethyl-N-2-propenyl-2-propenaminiumchlorid, 2-Propenamid | Merquat® S |
| Polyquaternium-10 | CAS 53568-66-4, 55353-19-0,54351-50-7, 68610-92-4, 81859-24-7 | Quaternäres Ammoniumsalz der Hydroxyethylcellulose | Celquat® SC-230M |
| Polyquaternium-11 | CAS 53633-54-8 | Vinylpyrrolidon/dimethylaminoethyl-Methacrylat-Copolymer/Diethylsulfat-Reaktionsprodukt | Gafquat®755N |
| Polyquaternium-16 | CAS 29297-55-0 | Vinylpyrrolidon/vinylimid azolinummethochlorid-Copolymer | Luviquat® HM552 |
| Polyquaternium-17 | CAS 90624-75-2 | | Mirapol®AD-1 |
| Polyquaternium-19 | CAS 110736-85-1 | Quaternisierter wasserlöslicher Polyvinylalkohol | |
| Polyquaternium-20 | CAS 110736-86-2 | In Wasser dispergierbarer quaternisierter Polyvinyloctadecylether | |
| Polyquaternium-21 | | Polysiloxanpolydimethyldimethylammoniumacetat-Copolymer | Abil® B 9905 |
| Polyquaternium-22 | CAS 53694-17-0 | Dimethyldiallylammoniumchlorid/Acrylsäure-Copolymer | Merquat®280 |
| Polyquaternium-24 | CAS 107987-23-5 | Polymeres quaternäres Ammoniumsalz der Hydroxyethylcellulose | Quartisoft® LM-200 |
| Polyquaternium-28 | CAS 131954-48-8 | Vinylpyrrolidon/Methacrylamidopropyltrimethylammoniumchlorid-Copolymer | Gafquat®HS-100 |
| Polyquaternium-29 | CAS 92091-36-6, 148880-30-2 | Chitosan, das mit Propylenoxid umgesetzt u. mit Epichlorhydrin quaternisiert wurde | Lexquat® CH |
| Polyquaternium-31 | CAS 136505-02-7, 139767-67-7 | Polymeres, quaternäres Ammoniumsalz, das durch die Umsetzung von PMAPA-Acrylate/Acrylsäure/Acrylonitrogens-Copolymeren u. Diethylsulfat hergestellt wird | Hypan® QT 100 |
| Polyquaternium-32 | CAS 35429-19-7 | N,N,N-Trimethyl-2-{[82-methyl-1-oxo-2-propenyl)oxy]- propenyl)oxy]ethanaminiumchlorid, polymer mit 2-Propenamid | |
| Polyquaternium-37 | CAS 26161-33-1 | | |
| Polyquaternium-44 | | Copolymeres quaternes Ammoniumsalz aus Vinylpyrrolidon und quaternisiertem Imidazolin | |

Weitere erfindungsgemäß vorteilhafte Filmbildner stellen Cellulosederivate und quaternisierte Guar Gum Derivate, insbesondere Guar Hydroxypropylammoniumchlorid (z.B. Jaguar Excel®, Jaguar C 162® der Firma Rhodia, CAS 65497-29-2, CAS 39421-75-5) dar.

Auch nichtionische Poly-N-vinylpyrrolidon/Polyvinylacetat-Copolymere (z.B. Luviskol VA 64W®, BASF), anionische Acrylat-Copolymere (z.B. Luviflex soft®, BASF), und/oder amphotere Amid/Acrylat/Methacrylat Copolymere (z.B. Amphomer®, National Starch) können erfindungsgemäß vorteilhaft als Filmbildner eingesetzt werden.

Erfindungsgemäß bevorzugte Filmbildner sind Polyquaternium-10, Polyquaternium-22 . und Polyquaternium-44 und Jaguar Excel®. Erfindungsgemäß besonders bevorzugt ist Polyquaternium-10 (z.B. Ucare Polymer JR-125® und Ucare Polymer JR-400®, Amerchol) in Kombination mit Jaguar Excel®.

Es ist erfindungsgemäß von Vorteil, die erfindungsgemäßen Tenside in einer Konzentration von 5 bis 30 Gewichts-%, bevorzugt in einer Konzentration von 10 bis 20 Gewichts-% und\ganz\ besonders bevorzugt in einer Konzentration von 12 bis 18 Gewichts-%, Jeweils bezogen auf das Gesamtgewicht der Zubereitung einzusetzen.

Endungsgemäß vorteilhaft können sowohl anionische, kationische, nichtionische und zwitterionische Tenside eingesetzt werden.

Vorteilhafte waschaktive anionische Tenside im Sinne der vorliegenden Erfindung sind Acylaminosäuren und deren Salze, wie
■ Acylglutamate, insbesondere Natriumacylglutamat
■ Sarcosinate, beispielsweise Myristoyl Sarcosin, TEA-lauroyl Sarcosinat, Natriumlauroylsarcosinat und Natriumcocoylsarkosinat,

Sulfonsäuren und deren Salze, wie
■ Acylisethionate, z.B. Natrium-/ Ammoniumcocoylisethionat,
■ Sulfosuccinate, beispielsweise Dioctylnatriumsulfosuccinat, Dinatriumlaurethsulfosuccinat, Dinatriumlaurylsulfosuccinat und Dinatriumundecylenamido MEA-Sulfosuccinat, Dinatrium PEG-5 Laurylcitratsulfosuccinat und Derivate,
sowie Schwefelsäureester, wie
■ Alkylethersulfat, beispielsweise Natrium-, Ammonium-, Magnesium-, MIPA-, TIPA-Laurethsulfat, Natriummyrethsulfat und Natrium C₁₂₋₁₃ Parethsulfat,
■ Alkylsulfate, beispielsweise Natrium-, Ammonium- und TEA- Laurylsulfat.

Vorteilhafte waschaktive kationische Tenside im Sinne der vorliegenden Erfindung sind quaternäre Tenside. Quaternäre Tenside enthalten mindestens ein N-Atom, das mit 4 Alkyl- oder Arylgruppen kovalent verbunden ist. Vorteilhaft sind beispielsweise Alkylbetain, Alkylamidopropylbetain und Alkylamidopropylhydroxysultain.

Vorteilhafte waschaktive amphotere Tenside im Sinne der vorliegenden Erfindung sind
■ Acyl-/dialkylethylendiamine, beispielsweise Natriumacylamphoacetat, Dinatriumacylamphodipropionat, Dinatriumalkylamphodiacetat, Natriumacylamphohydroxypropylsulfonat, Dinatriumacylamphodiacetat und Natriumacylamphopropionat,

Vorteilhafte waschaktive nicht-ionische Tenside im Sinne der vorliegenden Erfindung sind
■ Alkanolamide, wie Cocamide MEA/DEA/MIPA,
■ Ester, die durch Veresterung von Carbonsäuren mit Ethylenoxid, Glycerin, Sorbitan oder anderen Alkoholen entstehen,
■ Ether, beispielsweise ethoxylierte Alkohole, ethoxyliertes Lanolin, ethoxylierte Polysiloxane, propoxylierte POE Ether und Alkylpolyglycoside wie Laurylglucosid, Decylglycosid und Cocoglycosid.

Weitere vorteilhafte anionische Tenside sind:
■ Taurate, beispielsweise Natriumlauroyltaurat und Natriummethylcocoyltaurat,
■ Ether-Carbonsäuren, beispielsweise Natriumlaureth-13 Carboxylat und Natrium PEG-6 Cocamide Carboxylat, Natrium PEG-7-Olivenöl-Carboxylat
■ Phosphorsäureester und Salze, wie beispielsweise DEA-Oleth-10 Phosphat und Dilaureth-4 Phosphat,
■ Alkylsulfonate, beispielsweise Natriumcocosmonoglyceridsulfat, Natrium C₁₂₋₁₄ Olefinsulfonat, Natriumlaurylsulfoacetat und Magnesium PEG-3 Cocamidsulfat.

Weitere vorteilhafte amphotere Tenside sind
■ N-Alkylaminosäuren, beispielsweise Aminopropylalkylglutamid, Alkylaminopropionsäure, Natriumalkylimidodipropionat und Lauroamphocarboxyglycinat und N-Kokosfettsäureamidoethyl-N-hydroxyethylglycinat Natriumsalze und deren Derivate.

Weitere vorteilhafte nicht-ionische Tenside sind Alkohole.

Weitere geeignete anionische Tenside im Sinne der vorliegenden Erfindung sind ferner
■ Acylglutamate wie Di-TEA-palmitoylaspartat und Natrium Caprylic/ Capric Glutamat,
■ Acylpeptide, beispielsweise Palmitoyl hydrolysiertes Milchprotein, Natrium Cocoyl hydrolysiertes Soja Protein und Natrium-/ Kalium Cocoyl hydrolysiertes Kollagen
sowie Carbonsäuren und Derivate, wie
■ beispielsweise Laurinsäure, Aluminiumstearat, Magnesiumalkanolat und Zinkundecylenat,
■ Ester-Carbonsäuren, beispielsweise Calciumstearoyllactylat, Laureth-6 Citrat und Natrium PEG-4 Lauramidcarboxylat,
■ Alkylarylsulfonate.

Weitere geeignete kationische Tenside im Sinne der vorliegenden Erfindung sind ferner
■ Alkylamine,
■ Alkylimidazole,
■ ethoxylierte Amine
insbesondere deren Salze.

Weitere geeignete nicht-ionische Tenside im Sinne der vorliegenden Erfindung sind ferner Aminoxide, wie Cocoamidopropylaminoxid.

Es ist vorteilhaft das oder die erfindungsgemäßen waschaktiven Tenside aus der Gruppe der Tenside zu wählen, welche einen HLB-Wert von mehr als 25 haben, besonders vorteilhaft sind solche, welchen einen HLB-Wert von mehr als 35 haben.

Es ist erfindungsgemäß besonders bevorzugt, wenn als Tenside anionische, amphotere und/oder nichtionische Tenside eingesetzt werden wobei es ganz besonders bevorzugt ist, wenn als Tenside Natriumlaurylethersulfat, Cocoamidopropylbetain und/oder Dinatrium PEG-5 Laurylcitratsulfosuccinat und/oder N-Kokosfettsäureamidoethyl-N-hydroxyethylglycinat Natriumsalze eingesetzt werden.

Ferner können Polysorbate als waschaktive Agentien erfindungsgemäß vorteilhaft in die Emulsion eingearbeitet werden.

Erfindungsgemäß vorteilhaft sind Tensidkombinationen aus anionischen und zwitterionischen Tensiden.

Erfindungsgemäß besonders bevorzugt sind Tensidkombinationen aus Natriumlaurylethersulfat, Cocamidopropylbetain, Dinatriumcocoamphoacetat und/oder Dinatrium PEG-5 Laurylcitratsulfosuccinat, wobei eine Tensidkombination aus Natriumlaurylethersulfat, Cocamidopropylbetain und Dinatrium PEG-5 Laurylcitratsulfosuccinat ganz besonders bevorzugt ist. Erfindungsgemäß vorteilhaft enthält diese Tensidkombination Natriumlaurylethersulfat in einer Konzentration von 5 bis 11 Gewichts-%, Cocamidopropylbetain in einer Konzentration von 1 bis 5 Gewichts-%, Dinatrium PEG-5 Laurylcitratsulfosuccinat in einer Konzentration von 1 bis 5 Gewichts-%, jeweils bezogen auf das Gesamtgewicht der Zubereitung.

Vorteilhafte Ausführungsformen der endungsgemäßen kosmetischen Zubereitung sind auch dadurch gekennzeichnet, dass sie als weitere Bestandteile Trübungsmittel und/oder Perlglanzmittel enthalten. Als Trübungsmittel werden dabei erfindungsgemäß Substanzen und Substanzgemische verstanden, welche der Zubereitung ein trübes emulsionsartiges Aussehen verleihen.
Als Perlglanzmittel werden dabei erfindungsgemäß Substanzen und Substanzgemische verstanden, welche der Zubereitung ein opaleszierendes Aussehen verleihen. Erfindungsgemäß vorteilhaft ist es auch Mischungen aus Trübungs- und Periglanzmittel einzusetzen. Erfindungsgemäß vorteilhafte Trübungsmittel/Perlglanzmittel bzw. Mischungen sind unter anderem:
■ PEG-3 Distearat (z.B. CUTINA TS der Firma Cognis),
■ eine Kombination aus Glycoldistearat, Glycerin, Laureth-4 und Cocamidopropylbetain (z.B. Eüperlan PK 3000 und Euperlan PK 4000 der Firma Cognis),
■ eine Kombination aus Glycoldistearat, Cocosglucosiden, Glyceryloleat und Glycerylstearat (z.B. Lamesoft TM Benz der Firma Cognis).
■ Styrol/Acrylat Copolymere (z.B. Acusol OP 301 von Rohm & Haas)

Ferner ist es erfindungsgemäß von Vorteil, wenn in den erfindungsgemäßen Zubereitungen Salze eingesetzt werden. Diese führen zu einer Verdickung der Zubereitung, so dass Zubereitungen mit Viskositäten von 2000 mPas bis 6000 mPas hergestellt werden können. Unter Salzen sind ein- oder mehrwerteige Alkalimetallverbindungen bzw. Erdalkalimetallverbindungen mit Halogenanionen zu verstehen. Besonders bevorzugt wird Natriumchlorid eingesetzt.

Es ist erfindungsgemäß von Vorteil ein oder mehrere Alkylisalze bzw. Erdalkylisalze in einer Konzentration von 0,1 bis 3 Gewichts-%, bezogen auf das Gesamtgewicht der Zubereitung einzusetzen.

Bei mit Salzen schwer verdickbaren Zusammensetzungen ist es erfindungsgemäß von Vorteil, wenn in den erfindungsgemäßen Zubereitungen Verdicker eingesetzt werden. Diese können beispielsweise vorteilhaft aus weiteren Verbindungen der Gruppe der Gummen gewählt werden.

Zu den Gummen zählt man Pflanzen- oder Baumsäfte, die an der Luft erhärten und Harze bilden oder Extrakte aus Wasserpflanzen. Aus dieser Gruppe können vorteilhaft im Sinne der vorliegenden Erfindung gewählt werden beispielsweise Gummi Arabicum, Johannisbrotmehl, Tragacanth, Karaya, Guar Gummi, Pektin, Gellan Gummi, Carrageen, Agar, Algine, Chondrus, Xanthan Gummi.

Weiterhin vorteilhaft ist die Verwendung von derivatisierten Gummen wie z.B. Hydroxypropyl Guar (Jaguar® HP 8).

Unter den Polysacchariden und -derivaten befinden sich z.B. Hyaluronsäure, Chitin und Chitosan, Chondroitinsulfate, Stärke und Stärkederivate als erfindungsgemäß vorteilhafte Verdicker.

Unter den Cellulosederivaten befinden sich z.B. Methylcellulose, Carboxymethylcellulose, Hydroxyethylcellulose, Hydroxypropylmethylcellulose als erfindungsgemäß vorteilhafte Verdicker.

Unter den Schichtsilikaten befinden sich natürlich vorkommende und synthetische Tonerden wie z.B. Montmorillonit, Bentonit, Hektorit, Laponit, Magnesiumaluminiumsilikate wie Veegum®. Diese können als solche oder in modifizierter Form als Verdicker verwendet werden wie z. B. Stearylalkonium Hektorite.

Weiterhin können vorteilhaft auch Kieselsäuregele verwendet werden.

Unter den Polyacrylaten befinden sich z.B. Carbopol Typen der Firma Noveon (Carbopol 980, 981, 1382, 5984, 2984, ETD 2001, ETD 2020, ETD 2050 oder Pemulen TR1 & TR2).

Unter den Polymeren befinden sich z.B. Polyacrylamide (Seppigel 305), Polyvinylalkohole, PVP, PVP / VA Copolymere, Polyglycole.

Es ist erfindungsgemäß von Vorteil ein oder mehrere Verdicker in einer Konzentration von 0,01 bis 3 Gewichts-%, bevorzugt in einer Konzentration von 0,05 bis 2 Gewichts-% und ganz besonders bevorzugt in einer Konzentration von 0,1 bis 1 Gewichts-%, jeweils bezogen auf das Gesamtgewicht der Zubereitung einzusetzen.

Vorteilhaft, wenn auch nicht zwingend, können die erfindungsgemäßen Zubereitungen Konservierungsmittel enthalten.

Vorteilhafte Konservierungsmittel im Sinne der vorliegenden Erfindung sind beispielsweise Formaldehydabspalter (wie z. B. DMDM Hydantoin, welches beispielsweise unter der Handelsbezeichnung Glydant ^{™} Von der Fa. Lonza erhältlich ist), lodopropylbutylcarbamate (z. B. die unter den Handelsbezeichnungen Glycacil-L, Glycacil-S von der Fa. Lonza erhältlichen und/oder Dekaben LMB von Jan Dekker), Parabene (d. h. p-Hydroxybenzoesäurealkylester, wie Methyl-, Ethyl-, Propyl- und/oder Butylparaben), Phenoxyethanol, Ethanol, Benzoesäure und dergleichen mehr. Üblicherweise umfaßt das Konservierungssystem erfindungsgemäß ferner vorteilhaft auch Konservierungshelfer, wie beispielsweise Octoxyglycerin, Glycine Soja etc. Diese Liste der vorteilhaften Konservierungsmittel soll keineswegs limitierend sein. Vielmehr sind alle für Kosmetika oder Lebensmittel zugelassenden Konservierungsmittel vorteilhaft im Sinne der vorliegenden Erfindung.

Die wässrige Phase der erfindungsgemäßen Zubereitungen kann vorteilhaft übliche kosmetische Hilfsstoffe enthalten, wie beispielsweise Alkohole, insbesondere solche niedriger C-Zahl, vorzugsweise Ethanol und/oder Isopropanol, Diole oder Polyole niedriger C-Zahl sowie deren Ether, vorzugsweise Propylenglykol, Glycerin, Butylenglykol, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, Polymere, Schaumstabilisatoren, Elektrolyte sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z. B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole [von der Fa. Noveon], beispielsweise Carbopole der Typen 980, 981, 1382, 2984, 5984, ETD 2020, ETD 2050, Ultrez 10, jeweils einzeln oder in Kombination.

Die kosmetischen Zubereitungen gemäß der Erfindung können kosmetische Hilfsstoffe enthalten, wie sie üblicherweise in solchen Zubereitungen verwendet werden, z. B. Konservierungshelfery Komplexbildner, Antioxidantien, Puffer, Lösungsvermittler, Dispergiermittel, Bakterizide, Parfüme, Substanzen zum Verhindern oder Steigern des Schäumens, Farbstoffe, Pigmente, die eine färbende Wirkung haben, Verdickungsmittel, Perlglanzpigmente, weichmachende, anfeuchtende und/oder feuchhaltende Substanzen, Fette, Öle, Wachse oder andere übliche Bestandteile einer kosmetischen oder dermatologischen Formulierung wie Emulgatoren, Alkohole, Polyole, Polymere, Schaumstabilisatoren, Elektrolyte, organische Lösungsmittel oder Silikonderivate.

Die Zusammensetzungen enthalten gemäß der Erfindung außer den vorgenannten Substanzen gegebenenfalls die in der Kosmetik üblichen Zusatzstoffe, beispielsweise Parfüm, Farbstoffe, antimikrobielle Stoffe, rückfettende Agentien, Komplexierungs- und Sequestrierungsagentien, Perlglanzagentien, Antischuppenwirkstoffe, Pflanzenextrakte, Vitamine, Wirkstoffe.

Insbesondere ist es erfindungsgemäß von Vorteil der erfindungsgemäßen Zubereitung Vitamine, Pflanzenextrakte und UV-Lichtschutzfilter zuzusetzen. So ist beispielsweise der Zusatz von Calcium-Vitamin-Komplexen erfindungsgemäß besonders vorteilhaft.

Ferner ist es im Sinne der vorliegenden Erfindung, der erfindungsgemäßen Zubereitung Perlglanzpigmente, Glimmer und/oder Effektpigmente zuzusetzen, um die Zubereitung optisch attraktiver zu gestalten.

Die erfindungsgemäßen Zubereitungen stellen in jeglicher Hinsicht überaus befriedigende Präparate dar, welche nicht auf die in dieser Schrift erwähnte Rohstoffauswahl begrenzt sind:

Es ist vorteilhaft, wenn die erfindungsgemäße kosmetische Zubereitung in einer Flasche oder Quetschflasche aufbewahrt und aus dieser heraus angewendet werden.\ Entsprechend sind auch Flaschen oder Quetschflaschen, welche eine erfindungsgemäße Zubereitung enthalten, erfindungsgemäß.

Erfindungsgemäß vorteilhaft ist die Verwendung eines erfindungsgemäßen Haarreinigungsmittels als konditionierendes Haarwaschmittel (engt. Shampoo) für feines und/oder fettiges Haar.

Erfindungsgemäß ist ferner die Verwendung von Elastomeren in erfindungsgemäßen Haarreinigungsmitteln zur Regulierung der Menge an Filmbildner, die sich auf dem Haar ablagert.

Erfindungsgemäß ist nicht zuletzt die Verwendung von Elastomeren in erfindungsgemäßen Haarreinigungsmitteln zur Herstellung konditionierender Haarwaschmittel mit guten Griff- und Kämmeigenschaften und großem Haarvolumen für feines und/oder fettiges Haar.

Die folgenden Beispiele, in welchen die Waschpräparate zur Haarpflege beschrieben werden, sollen die erfindungsgemäßen Zusammensetzungen erläutern, ohne dass aber beabsichtigt ist, die Erfindung auf diese Beispiele zu beschränken. Die Zahlenwerte in den Beispielen bedeuten Gewichtsprozente, bezogen auf das Gesamtgewicht der Jeweiligen Zubereitungen.

### Beispiele

| | **1** | **2** | **3** | **4** | **5** | **6** |
|---|---|---|---|---|---|---|
| Natrium Laurylethersulfat | 9 | 9 | 9 | 9 | 9 | 11 |
| Cocamidopropyl Betain | 4 | 4 | 4 | 4 | 4 | 5 |
| Dinatrium PEG-5 Laurylcitrat Sulfosuccinat | 3 | 3 | 3 | 3 | 3 | - |
| Verdicker | 0.1 | 0.1 | 0.1 | 0.2 | 0,3 | 0.4 |
| Polyquaternium-10 | 0.3 | 0.1 | 0.2 | 0.3 | - | - |
| Guar Hydroxypropyl Trimonium Chlorid | - | - | - | 0.2 | 0.2 | - |
| PVP/VA Copolymer | - | - | - | - | - | 0.1 |
| Perlglanz | 1.5 | 4 | 4 | 2 | 2,5 | 1.5 |
| Elastomer | 0.1 | - 0.2 | 0.1 | 0.5 | 0.5 | 1.0 |
| PEG-40 hydriertes Rizinusöl | 0.2 | 0.2 | 0.2 | 0.2 | 0.2 | 0.2 |
| Natrium Salicylat | 0.4 | 0.4 | 0.4 | 0.4 | 0.4 | 0.4 |
| Natrium Benzoat | 0.4 | 0.4 | 0.4 | 0.4 | 0.4 | 0.4 |
| Parfüm | q.s. | q.s. | q.s. | q.s. | q.s. | q.s. |
| Wasser | ad 100 | ad 100 | ad 100 | ad 100 | ad 100 | ad 100 |

Beispiele 1 und 3 nicht erfindungsgemäss.

## Patentansprüche

1. Haarreinigungszubereitung enthaltend eine Kombination aus
a) einem oder mehreren Tensiden in einer Konzentration von 5 bis 30 Gew.-%,
b) einem oder mehreren Filmbildnern in einer Konzentration von 0,01 bis 5 Gew.-%,
c) dem Elastomeren Dimethicon/Vinyldimethicon Kreuzpolymeren (INCI: Dimethicone/Vinyl Dimethicone Crosspolymer) in einer Konzentration von 0,2 bis 2 Gew.-%,
jeweils bezogen auf das Gesamtgewicht der Zubereitung,
neben gegebenenfalls weiteren Wirk-, Hilfs- und Zusatzstoffen.

2. Haarreinigungsmittel nach Anspruch 1, **dadurch gekennzeichnet, dass** als Filmbildner Polyquaternium-2, Polyquaternium-5, Polyquaternium-6, Polyquatemium-7, Polyquaternium-10, Polyquatemium-11, Polyquaternium-16, Polyquaternium-17, Polyquaternium-19, Polyquaternium-20, Polyquaternium-21, Polyquaternium-22, Polyquaternium-24, Polyquaternium-28, Polyquaternium-29, Polyquaternium-31, Polyquaternium-32, Polyquaternium-37, Polyquaternium-44, nichtionische Poly-N-vinylpyrrolidon/Polyvinylacetat-Copolymere, anionische Acrylat-Copolymere, amphotere Amid/Acrylat/Methacrylat Copolymere, Cellulosederivate und quaternisierte Guar Gum Derivate eingesetzt werden.

3. Haarreinigungsmittel nach einem der Ansprüche 1 bis 2, **dadurch gekennzeichnet, dass** als Tenside eine Kombination aus anionischen und zwitterionischen Tensiden eingesetzt wird.

4. Haarreinigungsmittel nach einem der Ansprüche 1 bis 3, **dadurch gekennzeichnet, dass** ein oder mehrere Tenside gewählt werden aus der Gruppe Natriumlaurylethersulfat, Dinatriumcocoamphoacetat, Dinatrium PEG-5 Laurylcitratsulfosuccinat, Cocamidopropylbetain gewählt werden.

5. Haarreinigungsmittel nach einem der Ansprüche 1 bis 4, **dadurch gekennzeichnet, dass** es einen oder mehrere Verdicker in einer Konzentration von 0,01 bis 3 Gewichts-%, bezogen auf das Gesamtgewicht der Zubereitung enthält.

6. Haarreinigungsmittel nach einem der Ansprüche 1 bis 5, **dadurch gekennzeichnet, dass** Trübungs- und/oder Perlglanzmittel enthält.

7. Verwendung eines Haarreinigungsmittels nach einem der vorhergehenden Ansprüche als konditionierendes Haarwaschmittel für feines und/oder fettiges Haar.

8. Verwendung von Dimethicon/Vinyldimethicon Kreuzpolymeren (INCI: Dimethicone/Vinyl Dimethicone Crosspolymer) in Haarreinigungsmitteln nach einem der vorhergehenden Ansprüche zur Regulierung der Menge an Filmbildner, die sich auf dem Haar ablagert.

9. Verwendung von Dimethicon/Vinyldimethicon Kreuzpolymeren (INCI: Dimethicone/Vinyl Dimethicone Crosspolymer) in Haarreinigungsmitteln nach einem der vorhergehenden Ansprüche zur Herstellung konditionierender Haarwaschmittel mit guten Griff- und Kämmeigenschaften und großem Haarvolumen für feines und/oder fettiges Haar.

## Claims

1. Hair-cleansing preparation comprising a combination of
a) one or more surfactants in a concentration of from 5 to 30% by weight,
b) one or more film formers in a concentration of from 0.01 to 5% by weight,
c) the elastomer dimethicone/vinyldimethicone crosspolymer (INCI: Dimethicone/Vinyl Dimethicone Crosspolymer) in a concentration of from 0.2 to 2% by weight,
in each case based on the total weight of the preparation,
besides optionally further active ingredients, auxiliaries and additives.

2. Hair-cleansing composition according to Claim 1, **characterized in that** polyquaternium-2, polyquaternium-5, polyquaternium-6, polyquaternium-7, polyquaternium-10, polyquaternium-11, polyquaternium-16, polyquaternium-17, polyquaternium-19, polyquaternium-20, polyquaternium-21, polyquaternium-22, polyquaternium-24, polyquaternium-28, polyquaternium-29, polyquaternium-31, polyquaternium-32, polyquaternium-37, polyquaternium-44, nonionic poly-N-vinylpyrrolidone/polyvinyl acetate copolymers, anionic acrylate copolymers, amphoteric amide/acrylate/methacrylate copolymers, cellulose derivatives and quaternized guar gum derivatives are used as film formers.

3. Hair-cleansing composition according to one of Claims 1 to 2, **characterized in that** a combination of anionic and zwitterionic surfactants is used as surfactants.

4. Hair-cleansing composition according to one of Claims 1 to 3, **characterized in that** one or more surfactants are chosen from the group consisting of sodium lauryl ether sulphate, disodium cocoamphoacetate, disodium PEG-5 lauryl citrate sulphosuccinate, cocamidopropylbetaine.

5. Hair-cleansing composition according to one of Claims 1 to 4, **characterized in that** it comprises one or more thickeners in a concentration of from 0.01 to 3% by weight, based on the total weight of the preparation.

6. Hair-cleansing composition according to one of Claims 1 to 5, **characterized in that** it comprises opacifiers and/or pearlizing agents.

7. Use of a hair-cleansing composition according to one of the preceding claims as conditioning hair-washing composition for fine and/or greasy hair.

8. Use of dimethicone/vinyldimethicone crosspolymer (INCI: Dimethicone/Vinyl Dimethicone Crosspolymer) in hair-cleansing compositions according to one of the preceding claims for regulating the amount of film former which deposits on the hair.

9. Use of dimethicone/vinyldimethicone cross polymer (INCI: Dimethicone/Vinyl Dimethicone Crosspolymer) in hair-cleansing compositions according to one of the preceding claims for producing conditioning hair-washing compositions with good feel and combing properties and large hair volume for fine and/or greasy hair.

## Revendications

1. Nettoyant pour les cheveux comprenant une combinaison :
a) d'un ou de plusieurs tensioactifs en une concentration de 5 à 30 % en poids,
b) d'un ou de plusieurs agents filmogènes en une concentration de 0,01 à 5 % en poids,
c) du polymère croisé de diméthicone/vinyl-diméthicone élastomère (INCI : Dimethicone/ Vinyl Dimethicone Crosspolymer) en une concentration de 0,2 à 2 % en poids,
dans chaque cas par rapport au poids total de la préparation,
ainsi qu'éventuellement des ingrédients actifs, des auxiliaires et des additifs supplémentaires.

2. Composition nettoyante pour les cheveux selon la revendication 1, **caractérisée en ce que** l'on utilise, en tant qu'agent filmogène, le polyquaternium-2, le polyquaternium-5, le polyquaternium-6, le polyquaternium-7, le polyquaternium-10, le polyquaternium-11, le polyquaternium-16, le polyquaternium-17, le polyquaternium-19, le polyquaternium-20, le polyquaternium-21, le polyquaternium-22, le polyquaternium-24, le polyquaternium-28, le polyquaternium-29, le polyquaternium-31, le polyquaternium-32, le polyquaternium-37, le polyquaternium-44, des copolymères poly-N-vinyl-pyrrolidone/polyacétate de vinyle non-ioniques, des copolymères d'acrylate anioniques, des copolymères d'amide/acrylate/métacrylate amphotères, des dérivés de la cellulose et des dérivés de la gomme de guar quaternisés.

3. Composition nettoyante pour les cheveux selon l'une quelconque des revendications 1 à 2, **caractérisée en ce que** l'on utilise, en tant que tensioactifs, une combinaison de tensioactifs anioniques et zwitterioniques.

4. Composition nettoyante pour les cheveux selon l'une quelconque des revendications 1 à 3, **caractérisée en ce qu'**un ou plusieurs tensioactifs sont choisis parmi le groupe constitué du lauryléthersulfate de sodium, du cocoamphoacétate disodique, du laurylcitrate-sulfosuccinate de PEG-5 disodique et de la cocoamidopropylbétaïne.

5. Composition nettoyante pour les cheveux selon l'une quelconque des revendications 1 à 4, **caractérisée en ce qu'**elle comprend un ou plusieurs agents épaississants en une concentration de 0,01 à 3 % en poids par rapport au poids total de la préparation.

6. Composition nettoyante pour les cheveux selon l'une quelconque des revendications 1 à 5, **caractérisée en ce qu'**elle comprend des agents opacifiants et/ou des agents nacrants.

7. Utilisation d'une composition nettoyante pour les cheveux selon l'une quelconque des revendications précédentes en tant que composition lavante pour les cheveux, conditionnante pour des cheveux fins et/ou gras.

8. Utilisation de polymères croisés de diméthicone/ vinyldiméthicone (INCI : Dimethicone/Vinyl Dimethicone Crosspolymer) dans des compositions nettoyantes pour les cheveux selon l'une quelconque des revendications précédentes pour réguler la quantité d'agents filmogènes qui se déposent sur les cheveux.

9. Utilisation de polymères croisés de diméthicone/ vinyldiméthicone (INCI : Dimethicone/Vinyl Dimethicone Crosspolymer) dans des compositions nettoyantes pour les cheveux selon l'une quelconque des revendications précédentes pour produire des compositions lavantes pour les cheveux, conditionnantes avec de bonnes propriétés au toucher et au peignage et un grand volume de cheveux pour des cheveux fins et/ou gras.
